## Europäisches Patentamt
## European Patent Office
## Office européen des brevets

(11) Veröffentlichungsnummer: **0 022 425**
**A2**

(12) **EUROPÄISCHE PATENTANMELDUNG**

(21) Anmeldenummer: **80810195.0**

(22) Anmeldetag: **12.06.80**

(51) Int. Cl.³ **C 12 P 1/04**
C 12 P 21/00, C 07 G 11/00
A 61 K 35/74
//C12R1/01

(30) Priorität: **13.06.79 DE 2924006**

(43) Veröffentlichungstag der Anmeldung:
**14.01.81 Patentblatt 81'2**

(84) Benannte Vertragsstaaten:
**AT BE CH DE FR GB IT LI LU NL SE**

(71) Anmelder: **CIBA-GEIGY AG**
**Patentabteilung Postfach**
**CH-4002 Basel(CH)**

(71) Anmelder **Gesellschaft fur Biotechnologische**
**Forschung mbH (GBF)**
**Mascheroder Weg 1**
**D-3300 Braunschweig-Stöckheim(DE)**

(72) Erfinder **Augustiniak, Hermann, Dr.**
**In den Lindendöhren 19**
**D-3340 Wolfenbüttel(DE)**

(72) Erfinder: **Höfle, Gerhard, Dr.**
**Am Windmühlenberg 2**
**D-3300 Braunschweig(DE)**

(72) Erfinder: **Irschik, Herbert, Dr.**
**Alter Weg 56**
**D-3340 Wolfenbüttel(DE)**

(72) Erfinder: **Kemmer, Thorsten, Dr.**
**Schützenstrasse 2**
**D-3340 Wolfenbüttel(DE)**

(72) Erfinder: **Kunze, Brigitte, Dr.**
**Hinter Aegidien 5**
**D-3300 Braunschweig(DE)**

(72) Erfinder: **Reichenbach, Hans, Dr.**
**Platanenstrasse 35**
**D-3340 Wolfenbüttel(DE)**

(72) Erfinder **Reifenstahl, Günther, Dr.**
**Am Bülten 4 c**
**D-3300 Braunschweig(DE)**

(72) Erfinder **Trowitzsch, Wolfram, Dr.**
**Fuchstwete 10**
**D-3300 Braunschweig(DE)**

(72) Erfinder: **Gerth, Klaus, Dr.**
**Am Butterbusch 17**
**D-3300 Braunschweig(DE)**

(72) Erfinder: **Gruner, Johannes, Dr.**
**Buttiweg 23**
**CH-4113 Flüh(CH)**

(54) **Antibiotika, Verfahren und Zwischenprodukte zu deren Herstellung und deren Verwendung als pharmazeutische Präparate.**

(57) Gegenstand der Erfindung ist ein neues Antibiotikum-Gemisch, sowie dessen neue antibiotisch wirksame Einzel komponenten, und ihre Salze und Salzkomplexe. Das Antibiotikum-Gemisch entsteht bei der Fermentation eines neuen Stammes eines Mikroorganismus der Art Myxococcus fulvus aus der Familie der Myxococcaceae, welcher aus einer Bodenprobe isoliert wurde Die Fermentation erfolgt unter submersen aeroben Bedingungen in einem Kohlenstoff und Stickstoff sowie Mineralsalze enthaltenden wässerigen Nährmedium. Extrakte mit antibiotischer Aktivität können sowohl aus den Zellen wie aus dem Kulturüberstand gewonnen werden und aus diesen Extrakten lassen sich drei Einzelkomponenten als chemisch einheitliche Verbindungen isolieren, die als die Antibiotika A, B und C benannt werden Das Antibiotikum-Gemisch sowie seine Einzelkomponenten zeigen eine gute antibiotische Wirksamkeit gegenüber vers chiedenen Mikroorganismen.

EP 0 022 425 A2

./...

Croydon Printing Company Ltd

- 1 -

4-12871/GBF

Neue Antibiotika und fermentatives Verfahren zu ihrer Herstellung

Gegenstand der Erfindung ist ein neues Antibiotikum-Gemisch, sowie dessen neue antibiotisch wirksame Einzelkomponenten, und ihre Salze und Salzkomplexe, sowie pharmazeutische Präparate, die die genannten neuen Einzelkomponenten, oder ihre Salze und Salzkomplexe als Wirkstoffe enthalten, Verfahren zur Herstellung der neuen genannten Stoffe oder pharmazeutischen Präparate, sowie die Verwendung der neuen Stoffe oder pharmazeutischen Präparate als antibiotisch wirksame Mittel.

Die neuen antibiotisch wirksamen Stoffe entstehen bei der Fermentation eines neuen, unten identifizierten neuen Mikroorganismus. Die Erfindung betrifft somit auch diesen neuen Mikroorganismus, sowie die durch Züchtung desselben gewonnene Kulturbrühe und die daraus gewonnenen Extrakte, sowie die Verfahren zur Herstellung der Fermentationsbrühe und der Extrakte.

Herkunft und Hinterlegung des Mikroorganismus

Bei dem neuen Mikroorganismus handelt es sich um einen Stamm des Bakteriums Myxococcus fulvus der Klasse Flexibacteriae, der Ordnung Myxobacterales und der Familie Myxococcaceae. Der Stamm wurde im März 1969 aus einer Bodenprobe vom Kaiserstuhl (Baden/Bundesrepublik Deutschland) isoliert.

Der Stamm wurde am 28. März 1979 unter der Bezeichnung DSM 1525 bei der Deutschen Sammlung von Mikroorganismen/Göttingen hinterlegt.

Beschreibung des Stamms

Die vegetativen Zellen sind schlanke Stäbchen mit einer Länge von 3,5 bis 6 μm und einer Dicke von 0,8 μm und weisen leicht verjüngte Enden auf. Auf geeigneten Nährböden (wie Wasseragar mit Ausstrichen von Escherichia coli) bildet der Organismus rotbraune, tropfenförmige, weichschleimige Fruchtkörper mit einem Durchmesser von etwa 200 nm. In diesen Fruchtkörpern befinden sich kugelige, im Phasenkontrast stark lichtbrechende Myxosporen mit einem Durchmesser von 1,2 bis 1,5 μm. Das Bakterium bewegt sich gleitend fort. Die Kolonien bzw. Schwärme breiten sich deshalb allmählich über die Kulturplatten aus.

Der Organismus wächst auf Peptonagar, z.B.

- cy-agar: 0,3% Casitone (Difco); 0,1% Bäckerhefeextrakt (Difco); 0,1% $CaCl_2 \cdot 2H_2O$; 1,5% Agar; oder

- vy/2-agar: 0,5% Bäckerhefe (bezogen auf Frischgewicht); 0,1% $CaCl_2 \cdot 2H_2O$; 1,5% Agar; pH 7,2.

In Flüssigmedien wächst der Organismus in homogener Suspension, und zwar sowohl in Schüttelkolben (bei etwa 150 U/min), als auch in Fermentern. Als Medien lassen sich z.B. verwenden:

- 1% Pepton aus Casein, tryptisch verdaut (Merck/Darmstadt); 0,1% $MgSO_4 \cdot 7H_2O$; pH 7,2; oder
- 1% Pepton aus Casein, tryptisch verdaut (Merck/Darmstadt); 0,1% $MgSO_4 - 7H_2O$; 0,03% $CaCl_2 \cdot 2H_2O$; pH 7,0.

Die Kulturen lassen sich gut bei 30°C halten. Der Organismus wächst aerob. Die Generationszeit liegt bei 8,5 h ($\mu = 0,118$ $h^{-1}$). Die Ausbeuten liegen bei 5 g Zellmasse (Feuchtgewicht/l).

Der Organismus ist auf die Verwertung von Proteinen und Peptonen eingestellt und besitzt Enzyme zum Abbau anderer Mikroorganismen (Bakterien und Hefen).

Der Organismus lässt sich folgendermassen konservieren:

- Einfrieren vegetativer Zellen von Platten oder Flüssigkulturen in Pepton-Lösung bei -80°C;

- Trocknen von Fruchtkörpern auf Filterpapier; oder

- Trocknen von Myxosporen in Milch und Lagerung unter Stickstoff bei Zimmertemperatur.

## Kulturbrühe

Die Erfindung betrifft gemäss einer Ausführungsform eine Kulturbrühe von Myxococcus fulvus DSM 1525, die durch Kultivieren von Myxococcus fulvus DSM 1525 unter submersen aeroben Bedingungen in einem Kohlenstoff und Stickstoff sowie Mineralsalze enthaltenden wässerigen Nährmedium bei 15 bis 40°C erhältlich ist. Insbesondere ist die Kulturbrühe durch Kultivieren von Myxococcus fulvus 1525 bei 25 bis 35°C und vorzugsweise etwa 30°C erhältlich. Die Erfindung betrifft somit insbesondere auch diese Verfahren zur Herstellung der Kulturbrühe.

Die Ueberstände von Flüssigkulturen des Organismus zeigen antibiotische Aktivität gegen gram-positive Bakterien, wie Bacillus subtilis, Staphylococcus aureus.

Die antibiotische Aktivität kann z.B. gegen Staphylococcus aureus in 5o-ml-Schüttelkulturen schon während der logarithmischen Wachstumsphase ab einer optischen Dichte (gemessen bei lambda 623 nm und einem Lichtweg von 1 cm; $o.D._{623nm}$) von 0,4 nachgewiesen werden; die maximal erreichbare optische Dichte beträgt etwa 2. Die höchste Aktivitäten werden während der frühen stationären Phase erreicht. In Fermentern wird die Bildung der Aktivität durch hohen Sauerstoffpartialdruck unterdrückt.

Extrakte mit antibiotischer Aktivität, ein Antibiotikum-Gemisch und seine Einzelkomponenten können sowohl aus den Zellen als auch aus dem Kulturüberstand gewonnen werden.

Extrakt

So betrifft die Erfindung gemäss einer weiteren Ausführungsform einen Extrakt, der erhältlich ist durch: Abtrennen der Zellen von Myxococcus fulvus DSM 1525 von einer erfindungsgemäss erhaltenen Kulturbrühe,

(a) Extrahieren der abgetrennten Zellen mit einem Gemisch aus Wasser und einem polaren organischen Lösungsmittel und Abtrennen der extrahierten Zellen vom Extrakt, oder

(b) Extrahieren der abgetrennten Kulturbrühe mit einem mit Wasser nicht unbegrenzt mischbaren polaren organischen Lösungsmittel und Abtrennen der Extraktphase.

Bei der Variante (a) kann es sich im einen Extrakt handeln, der durch Extrahieren mit einem Gemisch aus Wasser und Keton, wie Aceton, oder $C_{1-3}$-Alkohol, wie Methanol, erhältlich ist, gegebenenfalls nach Waschen der Zellen mit einem Keton, z.B. Aceton. Bei der Variante (b) kann es sich um einen Extrakt handeln, der durch Extrahieren mit einem mit Wasser nicht unbegrenzt mischbaren Alkohol erhältlich ist, wie Butanol.

Die Erfindung betrifft insbesondere diese Verfahren zur Herstellung des Extrakts.

Antibiotikum-Gemisch

Die Erfindung betrifft gemäss einer weiteren Ausführungsform ein Antibiotikum-Gemisch, das aus den erfindungsgemässen Extrakten durch

(a) Behandeln des Extrakts mit einem Anionenaustauscher und Abtrennen des Anionenaustauschers vom flüssigen Medium,

(b) Chromatographieren des flüssigen Mediums an Aluminiumoxid und

(c) Gefriertrocknen erhältlich ist.

Insbesondere betrifft die Erfindung ein Wirkstoffgemisch, das aus dem aus Zellen gewonnenen Extrakt durch

(a) Abdestillieren des polaren organischen Lösungsmittels,

(b) Extraktion des wässerigen Mediums mit einem mit Wasser nicht unbegrenzt mischbaren polaren organischen Lösungsmittel und Trennen der beiden Phasen,

(c) Einengen der Lösungsmittelphase und Aufnehmen mit Wasser,

(d) Behandeln des wässerigen Mediums mit einem Anionenaustauscher und Abtrennen des Anionenaustauschers,

(e) Extrahieren des wässerigen Mediums mit einem mit Wasser nicht unbegrenzt mischbaren polaren organischen Lösungsmittel und Trennen der beiden Phasen,

(f) Einengen der Lösungsmittelphase,

(g) Chromatographieren der Lösungsmittelphase an Aluminiumoxid,

(h) Eluieren vom Aluminiumoxid mit einem Gemisch aus Wasser und Keton oder $C_{1-3}$-Alkohol,

(i) Abdestillieren des Ketons bzw. $C_{1-3}$-Alkohols,

(j) Extrahieren des wässerigen Mediums mit einem mit Wasser nicht unbegrenzt mischbaren polaren organischen Lösungsmittel und Trennen der beiden Phasen,

(k) Einengen der Lösungsmittelphase bis zur Trockne und Aufnahme in Wasser und

(l) Gefriertrocknen erhältlich ist.

Die Erfindung betrifft insbesondere auch ein Antibiotikum-Gemisch, das aus dem aus der Kulturbrühe gewonnenen Extrakt gemäss den vorstehenden Stufen (c) bis (l) erhältlich ist.

Vorzugsweise betrifft die Erfindung ein Antibiotikum-Gemisch, das durch Abdestillieren bzw. Einengen bei den vorstehend angeführten Stufen (a), (c), (f), (i) und (k) unter reduziertem Druck und/oder Verwendung von Alkohol, wie Butanol, als mit Wasser nicht unbegrenzt mischbarem polaren organischen Lösungsmittel gemäss den vorstehend angeführten Stufen (b), (e) und (j) erhältlich ist.

Dass es sich um dieselben Substanzen im Wirkstoffgemisch handelt, unabhängig davon, ob man von einem Zellextrakt oder von einem Kulturbrüheextrakt ausgeht, lässt sich daraus schliessen,
- dass in beiden Fällen die Reinigungsgänge und
- die $R_f$-Werte identisch sind und
- auf Dünnschichtplatten eine Fluoreszenzlöschung bei 354 nm auftritt.

## Charakterisierung des Antibiotika-Gemischs

Die folgenden Zusammenstellung zeigt das Wirkstoffspektrum beim Plättchentest. Dazu wird Material aus einer Chromatographie an Aluminiumoxid in Methanol bis zu einer Konzentration von 3 mg/ml gelöst. Diese  Stammlösung wird in Zweierschritten verdünnt. Von jeder Verdünnungsstufe werden 10 µl auf ein Filterplättchen übertragen, wobei die Aktivität beim Plättchentest anhand des Hemmhof-Durchmessers bestimmt wird.

| Testorganismus: | noch Hemmung bei angegebener Substanzmenge (µg/Plättchen) |
|---|---|
| a) Bakterien: | |
| Bacillus subtilis | + kleiner 9,8 |
| Staphylococcus aureus | + etwa 9,8 |
| Escherichia coli K12, Vollmedium | + 37,5 |
| Pseudomonas fluorescens | + 75 |
| b) Pilze: | |
| Mucor hiemalis | + 150 |
| C) Hefen | |
| Candida albicans | - |
| Schizosaccaromyces pombe | - |

- 7 -

Die im folgenden zusammengestellten minimalen Hemmkonzentrationen werden in Flüssigkulturen mit einem bis zu etwa 50%
angereicherten Wirkstoffgemisch getestet.

| Testorganismus | min. Hemmkonzentration ($\mu$g/ml) |
|---|---|
| Bacillus subtilis | 1 |
| Staphylococcus aureus | 1 |
| Streptococcus pyogenes Aronson | 2 |
| Streptococcus pneumoniae III | 0,5 |
| Streptococcus faecalis | 32 |
| Neisseria meningitidis | 2 |
| Neisseria gonorrhoeae | 0,5 |
| Escherichia coli K12 | 30 |
| Klebsiella pneumoniae | $\succ$128 |
| Serratia marcescens | 128 |
| Salmonella typhi | 128 |
| Proteus morganii | 128 |
| Proteus mirabilis | $\succ$ 128 |
| Pseudomonas aeruginosa | 32 |
| Pseudomonas fluorescens | 30 |
| Candida albicans | $\succ$ 128 |
| Schizosaccharomyces pombe | etwa 250 |

In vivo Versuche an der Maus mit dem gereinigten Antibiotikumgemisch ergeben folgende Resultate:

$ED_{50}$ bei Staphylococcus aureus   s.c.   2,3  mg/kg

"            "     p.o. $\succ$30    "

Streptococcus pyogenes Aronson   2 x 0,4  mg/kg Escherichia coli   s.c.   $\succ$2 x 10    "

Das oben genannte Antibiotikum-Gemisch zeigt ausserdem eine
gewisse anthelmintische Wirkung in vitro gegen Nematospiroides dubius.

Das neue Antibiotikum-Gemisch und seine Einzel-Komponenten
können zur Bekämpfung von Infektionen, die durch oben genannte sensible Organismen hervorgerufen werden, sowie als Futtermittelzusatz verwendet werden.

In Flüssigkulturen von Staphylococcus aureus lässt sich folgender Wirkungsmechanismus ermitteln. Das Wachstum des Bakteriums hört sofort nach Zugabe des Wirkstoffgemischs (3 µg/ml) auf. Der Einbau von $[^{14}C]$-Isoleucin wird nach Zugabe des Wirkstoffgemischs innerhalb von 4 min eingestellt. Der Einbau von $[^{14}C]$-Uracil sowie von $[^{14}C]$-Thymidin, sowie der Einbau von $[^{14}C]$-N-Acetylglucosamin in das Peptidoglycan verlangsamt sich etwa 20 min nach Zugabe des Wirkstoffgemischs.

Zur Stabilität des Wirkstoffgemischs lassen sich folgende Angaben machen:

(a) im Kulturüberstand (pH etwa 8) mindestens eine Stunde im kochenden Wasserbad;

(b) im Kulturüberstand (pH 2 bis 13) mindestens einen Tag bei Zimmertemperatur;

(c) nach Reinigung durch Chromatographie an Aluminiumoxid mindestens 9 Monate in wässeriger Lösung im Kühlschrank bei +4°C;

(d) nach Reinigung wie bei (c), jedoch in methanolischer Lösung, mindestens 4,5 Monate bei Zimmertemperatur.

## Antibiotikum-Einzelkomponenten

Schliesslich betrifft die Erfindung drei feste, farblose, als Antibiotika wirksame chemische Verbindungen, die als die Komponenten A, B und C des Antibiotikums gemäss der Erfindung charakterisiert werden, mit folgenden Spektrenpeaks: UV in Methanol (10 mg/l; lamda max. (nm) mit Extinktion E in Klammern):

für A: 222 (1,45) und 275 (0,06);

für B: 222 (2,02) und 275 (0,08);

für C: 222 (0,239) und 275 (0,01);

IR (KBr: Mx f 65-3; ʮ $(cm^{-1})$;

für C: 3600 bis 2700 (stark), 2965 (mittel), 2935 (mittel), 2875 (mittel), 1700 bis 1600 (sehr stark) und 1560 bis 1480 (stark); Fluoreszenzlöschung bei 254 nm bei A, B und C.

- 9 -

Die Verbindungen A, B und C besitzen gute Löslichkeit in Methanol und Butanol und geringe Löslichkeit in Wasser. In allen Verbindungen A, B und C lässt sich ein Peptidteil mit Arginin, Alanin und Valin im Verhältnis 1:2:3 nachweisen. Das Molekulargewicht von A und B ist kleiner als 1100 und dasjenige von C ist ca. 1100.

Die Abtrennbarkeit der neuen Verbindungen aus dem erfindungsgemässen Wirkstoffgemisch durch Chromatographie kann an einer Säule (substituiertes Kieselgel; Phasenumkehr) mit einem gepufferten Laufmittelgemisch aus Acetonitril/Isopropanol/Wasser/Triäthylamin/Ameisensäure (100:100:200:3:1) in der Reihenfolge A, B und C erfolgen. Die Verbindungen können aus den Eluaten durch Lyophilisation erhalten werden.

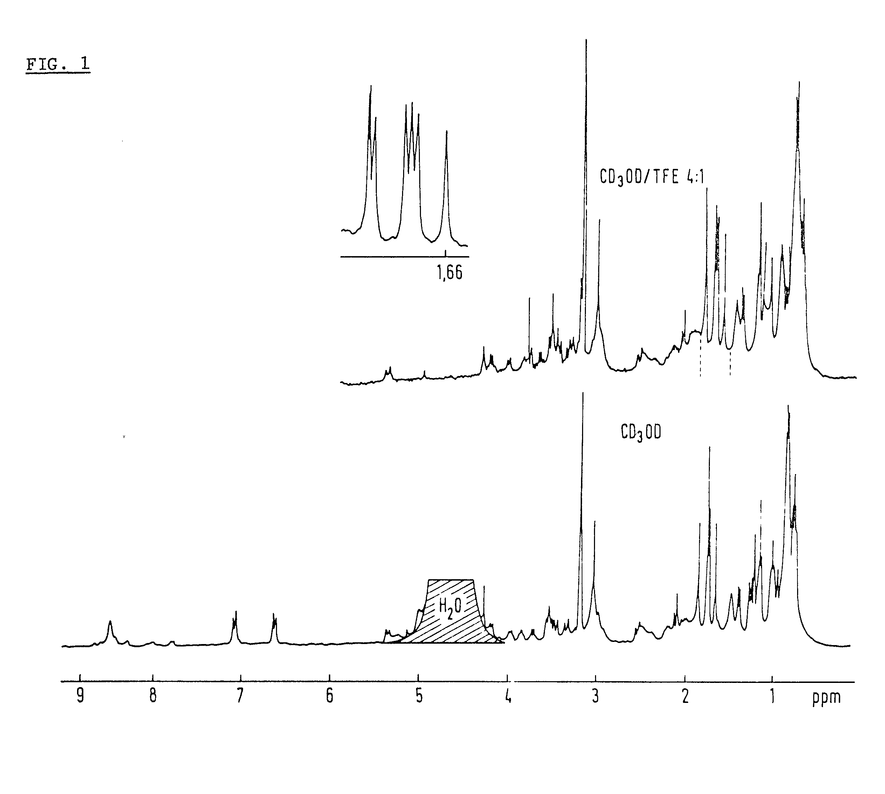

Das $^1$H-NMR-Spektrum (270 MHz; in $CD_3OD$ und $CD_3OD/CF_3COOD$ = 4:1) des Wirkstoffs C kann Figur 1 entnommen werden.

Das Molekulargewicht wurde aus dem Retentionsverhalten bei der Gelpermeationschromatographie an Sephasorb (Pharmacia AB) mit Methanol als Laufmittel abgeschätzt.

Alle drei Antibiotika A, B, C zeigen ein identisches Laufverhalten bei der Dünnschichtchromatographie. Es werden folgende $R_f$-Werte ermittelt:
(a) Kieselgelplatten mit Fluoreszenzindikator (Merck/Darmstadt):
- Propanol-2: Methanol: Wasser = 1:1:1; $R_f$ = 0,84;
- Butanol: Methanol: Wasser = 5:3:1; $R_f$ = 0,72;
(b) Aluminiumoxid-Platten mit Fluoreszenzindikator (Merck-Darmstadt):
- Propanol-2: Methanol: Wasser = 1:1:1, $R_f$ = 0,7 bis 0,75.

Alle drei Antibiotika A, B und C zeigen eine Fluoreszenzlöschung bei 254 nm. Aufgrund dieser Eigenschaft ist es dem Fachmann ohne weiteres möglich, die Wirksamkeit von Verfahrensmassnahmen zur Gewinnung des wirkstoffhaltigen erfindungsgemässen Extrakts, zur Gewinnung des Antibiotika-Gemischs und zur Gewinnung der einzelnen Komponenten zu beurteilen. Ausserdem lassen sich die Wirkstoffe an ihrer antibiotischen Aktivität erkennen. Diese ist bei allen drei Komponenten A, B, C grössenordnungsmässig etwa dieselbe.

Die Antibiotika A, B und C bilden Salze sowohl mit Metallen wie mit Säuren. Solche Salze können in an sich bekannter Weise hergestellt werden. Unter den Metallsalzen sind insbesondere diejenigen der Alkali- und Erdalkalimetalle zu erwähnen. Auch mit mehreren Schwermetallen bilden sie Metall - bzw. komplexe Salze, so z.B. mit Kupfer, Eisen, Kobalt, Mangan, Nickel und Zink.

Mit Säuren bilden die neuen Verbindungen Säureadditionssalze, unter denen besonders die pharmazeutisch verwendbaren, nichttoxischen zu erwähnen sind. Diese leiten sich z.B. von folgenden Säuren ab: anorganischen Säuren, z.B. Chlorwasserstoff-, Bromwasserstoff-, Salpeter-, Schwefel- oder Phosphorsäuren, oder organischen Säuren, wie organischen Carbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosslicylsäure, 2-Phenoxybenzoesäure, 2-Acetoxybenzoesäure, Embonsäure, Nicotinsäure oder Isonicotinsäure, oder organischen Sulfonsäuren, z.B. Methansulfonsäure, Aethansulfonsäure, 2-Hydroxy-äthansulfonsäure, Aethan-1,2-disulfonsäure, Benzolsulfonsäure, p-Toluolsulfonsäure oder Naphthalin-2-sulfonsäure. Andere Säureadditionssalze können · z.B. als Zwischenprodukte, z.B. zur Reinigung der freien Verbindungen oder in der Herstellung von anderen Salzen, sowie zur Charakterisierung verwendet werden, wie z.B. solche mit Pikrin-, Pikrolon-, Flavian-, Phosphorwolfram-, Phosphormolybdän-, Chlorplatin-, Reinecke- oder Perchlorsäure.

Die oben genannten Säureadditionssalze sind meistens in Wasser gut löslich und eignen sich daher besonders gut zur Herstellung von Injektionspräparaten.

Die vorliegende Erfindung betrifft ebenfalls pharmazeutische Präparate, welche eines oder mehrere der Antibiotika gemäss der Erfindung oder ihre pharmazeutisch verwendbaren Salze, Komplex-Salze oder Depot-Formen, als Wirksubstanz enthalten, sowie Verfahren zu deren Herstellung. Bei den erfindungsgemässen pharmazeutischen Präparaten handelt es sich um solche zur topischen, oralen oder rektalen oder insbesondere parenteralen Verabreichung, welche den pharmakologischen Wirkstoff allein oder zusammen mit einem entsprechenden pharmazeutisch anwendbaren Trägermaterial enthalten.

Vorzugsweise verwendet man die pharmakologisch wirksamen Verbindungen der vorliegenden Erfindung in Form von parenteral, z.B.intramuskulär oder subcutan, verabreichbarer Präparate oder von Infusionslösungen. Solche Präparate bzw. Lösungen sind vorzugsweise isotonische wässrige Lösungen oder Suspensionen, wie ölige Injektionssuspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Wässrige Injektionssuspensionen können mit viskositätserhöhenden Stoffen, z.B. Natrium-Carboxymethylcellulose, Sorbit und/oder Dextran und gegebenenfalls Stabilisatoren versehen sein. Die vorliegenden pharmazeutischen Präparate, die, wenn erwünscht, weitere pharmakologisch wertvolle Stoffe enthalten können, werden in an sich bekannter Weise, z.B. mittels konventioneller Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes.

Ausser den parenteral verabreichbaren pharmazeutischen Präparaten kommen auch solche zur enteralen,z.B.oralen, Verabreichung an Warmblüter in Frage, welche einen Gehalt von etwa 10% bis etwa 90%, insbesondere 20-75%,des pharmakologischen Wirkstoffs allein oder zusammen mit einem pharmazeutisch anwendbaren Trägermaterial aufweisen und sind Präparate in Dosiseinheitsform, wie Dragées, Tabletten, Kapseln, Suppositorien oder Ampullen. Sie werden in an sich bekannter Weise, z.B. mittels konventioneller Misch-, Granulier-, Dragier-, Lösungs- oder Lyophilisierungsverfahren hergestellt.

Geeignete Trägerstoffe für Tabletten und/oder Dragées sind insbesonder Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke,Gelatine,Tragacanth, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk,Stearinsäure oder Salze davon, wie Magnesiumoder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemisches oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffe beigefügt werden.

Weitere, oral anwendbare pharmazeutische Präparate sind Steckkapseln aus Gelatine, sowie weiche, geschlossene Kapseln aus Gelatine
und einem Weichmacher, wie Glycerin oder Sorbitol.

Die Erfindung betrifft sodann insbesondere die oben beschriebenen Verfahren zur Herstellung des Antibiotikum - Gemisches und seiner
Komponenten A, B und C, und Ausführungsformen, bei denen auf einer bestimmten Stufe unterbrochen wird oder man die fehlenden Schritte ausführt.

Die Erfindung umfasst ebenfalls die Verwendung der neuen Antibiotika oder ihrer pharmazeutisch verwertbaren Salze als pharmakologisch
wirksame Stoffe, vorzugsweise in Form von pharmazeutischen Präparaten.
Die Dosierung des Wirkstoffes hängt von der Warmblüter-Spezies, dem
Körpergewicht und Alter und vom individuellen Zustand, sowie von der
Applikationsart ab. Durchschnittlich wird einem Warmblüter von etwa
70 kg Körpergewicht eine Tagesdosis im Bereich zwischen 3 mg und
3000 mg, vorzugsweise zwischen 30 mg und 600 mg, Wirkstoff verabreicht.

Die neuen Antibiotika können auch in der Tiermedizin verwendet
werden.

Nachstehend wird die Erfindung durch Beispiele näher erläutert.

## Beispiel 1: Fermentation im Fermenter

Es wird ein 70-l-Fermenter mit einem Arbeitsvolumen von 70 l
verwendet. Das Medium enthält in Leitungswasser 1 % Pepton aus Casein
(tryptisch verdaut; Merck/Darmstadt), 0,1 % $MgSO_4 \cdot 7H_2O$, 0,03 % $CaCl_2 \cdot 2H_2O$
und 0,03 % Antischaum N 115 (Brenntag/Mülheim/Ruhr) bei pH 7.0. Der Fermenter wird durch Autoklavieren stabilisiert. Das Calciumchlorid wird
erst mit dem Inoculum zugegeben. Das Animpfvolumen von 6 l aus Schüttelkulturen ergibt eine optische Anfangsdichte (o.D.$_{623nm}$) von 0,35.
Die Temperatur beträgt 30°C. Es wird mit 500 Umdrehungen/min
(Umwurf) gerührt. Die Belüftung beträgt etwa 0.12 Nl/min und 1 Kulturvolumen, wobei der Sauerstoffgehalt 24 h lang bei einer Sättigung von
etwa 85 bis 90% gehalten wird. Nach 24 h wird durch Reduzieren der
Luftzufuhr der Sauerstoffgehalt auf etwa 80 bis 85% gesenkt. Danach
sinkt er kontinuierlich im Verlauf weiterer 24 h auf etwa 15%. Die
Gesamtfermentationsdauer beträgt 48 h. Es werden eine optische Enddichte von 2,1 und ein End-pH von 7,9 erreicht, wobei der pH nicht
reguliert wird.

Gewinnung des Antibiotikum-Gemisches aus Kulturüberstand

Zellen und Kulturflüssigkeit werden in einer Durchlaufzentrifuge getrennt. Die Kulturflüssigkeit wird mit einem halben
Volumen Butanol ausgeschüttelt (bzw. in einem Parallelversuch mit
einem drittel Volumen Butanol in einer Gegenstromanlage extrahiert).
Die Butanolphase wird unter reduziertem Druck bei 40°C eingeengt,
bis eine ölige Flüssigkeit zurückbleibt. Diese wird in 2,0 bis
2,5 l destilliertem Wasser aufgenommen. Zu dieser Lösung gibt man
500 g Ionenaustauscher Dowex 1 X 2 (50 bis 100 Maschen) und rührt
1 h lang. Der Ionenaustauscher ist zuvor mit Wasser gewaschen worden.
Anschliessend wird das Austauscherharz auf einem Büchner-Trichter
abgetrennt und mit Wasser gewaschen, bis ein Endvolumen von 3 l
erreicht wird. Die schwach grünlich-gelb gefärbte Wasserphase enthält die Aktivität. Sie wird zweimal mit jeweils einem halben Volumen
Butanol ausgeschüttelt. Die Butanolphase wird wie zuvor angegeben
bis zu einem Volumen von etwa 30 ml eingeengt. Diese butanolische
Lösung wird an 300 g Aluminiumoxid (Aktivität 1; Woelm/Eschwege)
chromatographiert. Die beladene Säule wird zunächst mit Butanol und
danach mit Butanol/Methanol gewaschen. Anschliessend wird die Aktivität mit Methanol und Wasser von der Säule eluiert. Das Methanol
wird aus dem Eluat unter reduziertem Druck bei 40°C abdestilliert.

Anschliessend wird die wässerige Phase zweimal mit einem halben
Volumen Butanol extrahiert. Die Butanolphasen werden vereinigt und zur
Trockne eingeengt. Der Rückstand wird in etwa 50 ml Wasser aufgenommen
und gefriergetrocknet. Das anfallende weisse Pulver wird bei -30°C
aufbewahrt.

Die Ausbeute beträgt bis einschliesslich der Chromatographie
an Aluminiumoxid 800 mg Rohprodukt. Das entspricht etwa 400 mg Antibiotikum-Gemisch = 5,7 mg/l Kulturvolumen.

Isolierung der einzelnen Antibiotika

Zur weiteren Reinigung wird das erhaltene Rohgemisch in
einem Hochdruckflüssigchromatographen an einer präparativen Säule
[(RP-18:Silikagel mit an der Oberfläche über Si-C-Bindungen kovalent
gebundenen Alkylgruppen mit $C_{18}$-Alkylkette (Merck)]mit dem gepufferten
Laufmittel Acetonitril/Isopropanol/Wasser/Triäthylamin/Ameisensäure
(100:100:200:3:1) chromatographiert. Dabei fallen drei Fraktionen an,
die eine Fluoreszenzlöschung bei 254 nm zeigen. Aus diesen drei
Fraktionen werden durch Gefriertrocknen farblose, amorphe Antibiotika
A, B bzw. C erhalten. Die Ausbeuten betragen (bezogen auf eingesetztes
Rohgemisch): 0,7% für A; 0,7% für B; 50,0% für C.

Beispiel 2: Es wird Beispiel 1 mit der Ausnahme wiederholt, dass das
Wirkstoffgemisch aus Zellen gewonnen ist. Dazu werden 5 g gefriergetrocknete Zellen mit 200 ml Aceton 2 h lang verrührt und danach
durch Zentrifugieren abgetrennt. Der Ueberstand enthält keine Aktivitäten und wird verworfen. Die sedimentierten Zellen werden mit 300
ml Aceton/Wasser (2:8 auf Volumenbasis) 1 h lang gerührt. Die Zellen
werden durch Zentrifugieren abgetrennt und das Aceton wird aus dem
Ueberstand durch Destillieren entfernt. Die zurückbleibende wässerige
Phase wird mit einem halben Volumen Butanol extrahiert. Die Butanollösung wird gemäss Beispiel 1 weiterverarbeitet.

- 16 -

Beispiel 3: Fermentation im Schüttelkolben

Es wird ein Medium von pH 7,0 verwendet, welches 1 % Pepton aus Casein (tryptisch verdaut; Merck/Darmstadt) und 0,1 % $MgSO_4 \cdot 7H_2O$ in Leitungswasser enthält. 500 ml Medium werden in einem 1-1-Erlenmeyerkolben aus einer Vorkultur zu 5 Volumenprozent beimpft. Die optische Anfangsdichte beträgt 0,2. Nach 70-stündiger Kultivierung bei 30°C wird eine optische Dichte von 1,88 bei einem pH von 7,6 erreicht. Unter diesen Kulturbedingungen beträgt die Generationszeit während der ersten 20 h 8,5 h. Danach verlangsamt sich das Wachstum allmählich auf eine Generationszeit von etwa 20 h.

Gewinnung des Antibiotikum-Gemisches aus Kulutrüberstand

Die Gewinnung erfolgt gemäss Beispiel 1. Die Ausbeute beträgt nach der Säulenchromatographie an Aluminiumoxid 8,8 mg Rohprodukt/1, was etwa 4,4 mg Wirkstoffgemisch/1 Kulturüberstand entspricht.

Patentansprüche

1. Antibiotikum-Gemisch enthalten in einer Kulturbrühe von Myxococcus fulvus DSM 1525, die durch Kultivieren von Myxococcus fulvus DSM 1525 unter submersen aeroben Bedingungen in einem Kohlenstoff und Stickstoff sowie Mineralsalze enthaltenden wässrigen Nährmedium bei 15° bis 40° erhältlich ist, seine Salze und Salzkomplexe.

2. Die Komponente A des Antibiotikums gemäss Anspruch 1, welche eine feste, farblose Verbindung mit folgenden Eigenschaften darstellt: UV-Spektrum in Methanol (10mg/l ; lambda max. (nm) mit Extinktion E in Klammern 222(1,45) und 275 (0,06); Fluoreszenzlöschung bei 254 nm; gute Löslichkeit in Methanol und Butanol und geringe Löslichkeit in Wasser; Anwesenheit eines Peptidteils mit Arginin, Alanin und Valin im Verhältnis 1:2:3; Molekulargewicht von kleiner als 1100, und ihre Salze und Salzkomplexe.

3. Die Komponente B des Antibiotikums gemäss Anspruch 1, welche eine feste, farblose Verbindung mit folgenden Eigenschaften darstellt: UV-Spektrum in Methanol (10 mg/l; lambda max. (nm) mit Extinktion E in Klammern) 222 (2,02) und 275 (0,08); Fluoreszenzlöschung bei 254 nm; gute Löslichkeit in Methanol und Butanol und geringe Löslichkeit in Wasser; Anwesenheit eines Peptidteils mit Arginin, Alanin und Valin im Verhältnis 1:2:3; Molekulargewicht von kleiner als 1100, und ihre Salze und Salzkomplexe.

4. Die Komponente C des Antibiotiums gemäss Anspruch 1, welche eine feste, farblose Verbindung mit folgenden Eigenschaften darstellt: UV-Spektrum in Methanol (10 mg/l; lambda max. (nm) mit Extinktion E in Klammern 222 (0,239) und 275) (0,01); IR-Spektrum [(KBr: Mx f 65-3; ny (cm$^{-1}$)]: 3600 bis 2700 (stark), 2965 (mittel), 2935 (mittel), 2875 (mittel), 1700 bis 1600 (sehr stark) und 1560 bis 1480 (stark); Fluoreszenzlöschung bei 254 nm; gute Löslichkeit in Methanol und Butanol und geringe Löslichkeit in Wasser; Anwesenheit eines Peptidteils mit Arginin, Alanin und Valin im Verhältnis 1:2:3;

- 18 -

Molekulargewicht von ca. 1100,
und ihre Salze und Salzkomplexe.

5.　　Verfahren zur Herstellung einer Kulturbrühe enthaltend das
Antibiotikum-Gemisch gemäss Anspruch 1, dadurch gekennzeichnet, dass
man Myxococcus fulvus DSM 1525 unter submersen aeroben Bedingungen in
einem Kohlenstoff oder Stickstoff sowie Mineralsalze enthaltenden
wässrigen Nährmedium bei 15 bis 40° kultiviert.

6.　　Verfahren nach Anspruch 5, dadurch gekennzeichnet, dass man
die Kultivierung bei 25° - 35°, insbesondere bei ca. 30° durchführt.

7.　　Kulturbrühe erhältlich nach einem der Ansprüche 5 oder 6.

8.　　Extrakt enthaltend das Antibiotikum-Gemisch gemäss Anspruch 1,
erhältlich durch Abtrennen der Zellen von Myxococcus fulvus DSM 1525
von gemäss Anspruch 5 oder 6 erhaltener Kulturbrühe und

(a) Extrahieren der abgetrennten Zellen mit einem Gemisch aus Wasser
    und einem polaren organischen Lösungsmittel und Abtrennen der
    extrahierten Zellen vom Extrakt oder

(b) Extrahieren der abgetrennten Kulturbrühe mit einem mit Wasser nicht
    unbegrenzt mischbaren polaren organischen Lösungsmittel und Abtrennen der Extraktphase.

9.　　Extrakt nach Anspruch 8 (a), dadurch gekennzeichnet, dass er durch
Extrahieren mit einem Gemisch aus Wasser und Keton, z.B. Aceton, oder
$C_{1-3}$-Alkohol, z.B. Methanol, erhältlich ist, gegebenenfalls nach
Waschen der Zellen mit einem Keton, z.B. Aceton.

10.　　Extrakt nach Anspruch 8 (b), dadurch gekennzeichnet, dass er
durch Extrahieren mit einem mit Wasser nicht unbegrenzt mischbaren
Alkohol, z.B. Butanol, erhältlich ist.

11.　　Verfahren zur Herstellung eines Extraktes gemäss einem der

- 19 -

Ansprüche 8 bis 10, dadurch gekennzeichnet, dass man die Zellen von Myxococcus fulvus DSM 1525 aus einer Kulturbrühe gemäss Anspruch 7 abtrennt und

(a) die abgetrennten Zellen mit einem Gemisch aus Wasser und einem polaren organischen Lösungsmittel extrahiert und die extrahierten Zellen vom Extrakt abtrennt oder

(b) die abgetrennte Kulturbrühe mit einem mit Wasser nicht unbegrenzt mischbaren polaren organischen Lösungsmittel extrahiert und die Extraktphase abtrennt.

12.    Verfahren gemäss Anspruch 11 (a), dadurch gekennzeichnet, dass man mit einem Gemisch aus Wasser und Keton, z.B. Aceton, oder $C_{1-3}$-Alkohol, z.B. Methanol, extrahiert, gegebenenfalls nach Waschen der Zellen mit einem Keton, z.B. Aceton.

13.    Verfahren nach Anspruch 11 (b), dadurch gekennzeichnet, dass man mit einem mit Wasser nicht unbegrenzt mischbaren Alkohol extrahiert, z.B. Butanol.

14.    Verfahren zur Herstellung des Antibiotikum-Gemisches gemäss Anspruch 1, dadurch gekennzeichnet, dass man den Extrakt gemäss Anspruch 8 (a), Anspruch 8 (b), Anspruch 9 oder Anspruch 10

(a) mit einem Anionenaustauscher behandelt und den Anionenaustauscher vom flüssigen Medium abtrennt,
(b) das flüssige Medium an Aluminiumoxid chromatographiert und
(c) gefriertrocknet.

15.    Verfahren nach Anspruch 14, dadurch gekennzeichnet, dass man aus dem Extrakt gemäss Anspruch 8 (a) oder Anspruch 9
(a) das polare organische Lösungsmittel abdestilliert,
(b) das wässerige Medium mit einem mit Wasser nicht unbegrenzt mischbaren polaren organischen Lösungsmittel,[wie einem Alkohol] extrahiert und die beiden Phasen trennt,

(c) die Lösungsmittelphase einengt und mit Wasser aufnimmt,

(d) das wässerige Medium mit einem Anionenaustauscher behandelt und den Anionenaustauscher abtrennt,

(e) das wässerige Medium mit einem mit Wasser nicht unbegrenzt mischbaren polaren organischen Lösungsmittel [wie einem Alkohol], extrahiert und die beiden Phasen trennt,

(f) die Lösungsmittelphase einengt,

(g) die Lösungsmittelphase an Aluminiumoxid chromatographiert,

(h) vom Aluminiumoxid mit einem Gemisch aus Wasser und Keton oder $C_{1-3}$-Alkohol eluiert,

(j) das wässerige Medium mit einem mit Wasser nicht unbegrenzt mischbaren polaren organischen Lösungsmittel [wie einem Alkohol] extrahiert und die beiden Phasen trennt,

(k) die Lösungsmittelphase bis zur Trockne einengt und in Wasser aufnimmt und

(1) gefriertrocknet.


16. Verfahren nach Anspruch 14, dadurch gekennzeichnet, dass man den Extrakt gemäss Anspruch 8 (b) oder Anspruch 10 gemäss Anspruch 15 (c) bis (1) aufarbeitet.


17. Verfahren zur Herstellung eines der Antibiotikums-Komponenten A, B oder C, nach einem der Ansprüche 2-4, dadurch gekennzeichnet, dass man das durch Ausübung eines der in den Ansprüchen 14-17 beanspruchten Verfahren erhältliche Antibiotikum-Gemisch an einer Säule (substituiertes Kieselgel; Phasenumkehr) mit einem gepufferten Laufmittelgemisch aus Acetonitril/Isopropanol/Wasser/Triäthylamin/Ameisensäure (100:100:200:3:1) chromatographiert, als Fraktionen in der Reihenfolge Komponente A, Komponente B und Komponente C abtrennt und danach durch Abdampfen des Lösungsmittelgemisches gewinnt.


18. Pharmazeutische Präparate enthaltend ein Gemisch oder eine Verbindung gemäss einem der Ansprüche 1-4.

19.    Eine Verbindung oder ein Gemisch gemäss einem der Ansprüche 1-4, zur Anwendung in einem Verfahren für die therapeutische Behandlung des tierischen oder menschlichen Körpers.

20.    Die Anwendung eines Gemisches oder einer Verbindung gemäss einem der Ansprüche 1-4 als Antibiotikum.

21.    Der neue Mikroorganismus-Stamm Mixococcus fulvus DSM 1525.

FIG. 1

CD$_3$OD/TFE 4:1

1,66

CD$_3$OD

H$_2$O

9    8    7    6    5    4    3    2    1    ppm

1/1

0022425